# EUROPÄISCHE PATENTSCHRIFT

(11) **EP 2 460 492 B1**
(45) Veröffentlichungstag und Bekanntmachung des Hinweises auf die Patenterteilung: **28.08.2013**
(21) Anmeldenummer: 11191590.6
(22) Anmeldetag: 01.12.2011
(51) Int. Cl.: A61F 2/42, A61F 2/30

(54) **Handgelenkprothese**
Wrist prosthesis
Prothèse pour poignet

(30) Priorität: 02.12.2010 DE 102010053265
(43) Veröffentlichungstag der Anmeldung: 06.06.2012
(73) Patentinhaber: Lazic, Voja, 5000 Aarau (CH)
(72) Erfinder: Lazic, Voja, Dr., 5000 Aarau (CH); Riner, Marc. A., Dr., 5000 Aarau (CH); Ingold, Stephan, 4654 Lostorf (CH)
(74) Vertreter: Popp, Eugen

(56) Entgegenhaltungen:
- FR-A1- 2 801 194
- US-A1- 2006 161 260
- US-A1- 2008 027 558
- US-A1- 2009 319 050
- US-B1- 6 436 146

## Beschreibung

Die Erfindung betrifft eine Handgelenkprothese nach dem Oberbegriff des Patentanspruchs 1.

Handgelenkprothesen sind seit einiger Zeit bekannt und ersetzen, beispielsweise im Fall einer Arthrose, das Handgelenk, wobei durch die Handgelenkprothese eine Verbindung zwischen dem Radius, also der Speiche, und Handwurzelknochen hergestellt wird. Zu diesem Zweck sind bislang übliche Handgelenkprothesen, wie beispielsweise in der US 2008/0027558 A1 beschrieben, modular aufgebaut, wobei eine proximale Prothesenkomponente im Radius und eine distale Prothesenkomponente in der Mittelhand, genauer in den Mittelhandknochen verankert wird. Die US 2009/0319050 zeigt die Merkmale des Oberbegriffs von Anspruch 1.

Diese bislang bekannten Handgelenkprothesen weisen jedoch zahlreiche Nachteile auf, die zum einen darin bestehen, dass sowohl die proximale als auch die distale Komponente dieser bisherigen Handgelenkprothesen sehr exakt und paßgenau fixiert werden müssen, um eine Funktionsfähigkeit des Handgelenks zu gewährleisten. Diese paßgenaue Fixierung ist jedoch äußerst schwierig durchzuführen, da die einzelnen Handwurzel- und Mittelhandknochen gegeneinander bewegbar sind und darüber hinaus nur äußerst wenig Knochensubstanz aufweisen, in der eine Schraube oder ein Nagel sicher verankert werden könnte. Darüber hinaus ist die distale Komponente herkömmlicher Handgelenkprothesen als starres Element ausgebildet, das in mehreren und insbesondere verschiedenen Handwurzel- oder Mittelhandknochen verankert werden muss, so dass diese Handwurzel- und Mittelhandknochen gegeneinander durch das distale Handgelenkprothesenelement versteift werden, so dass das Handgelenk nach erfolgter Implantation bisheriger Handgelenkprothesen zumindest teilweise versteift ist und an Beweglichkeit einbüßt. Ferner ist der operative Aufwand beim Implantieren bislang bekannter Handgelenkprothesen aufgrund der Notwendigkeit der vorerwähnten vielfachen Fixierungen in den Handwurzel- und Mittelhandknochen einerseits und der Speiche andererseits groß, wobei zusätzlich ein erheblicher Eingriff in die Bindegewebs- und Bänderstruktur der zu operierenden Hand mit entsprechenden möglichen Komplikationen notwendig ist.

Der Erfindung liegt die Aufgabe zugrunde, eine Handgelenkprothese zur Verfügung zu stellen, die oben genannte Nachteile nicht aufweist, relativ einfach zu implantieren und sicher zu verankern ist, als Spacer (Platzhalter) dient und darüber hinaus die natürliche Beweglichkeit des Handgelenks weitestgehend erhält.

Diese Aufgabe wird durch eine Handgelenkprothese nach Patentanspruch 1 gelöst.

Insbesondere wird die Aufgabe durch eine Handgelenkprothese mit einer Radialkomponente zur Befestigung am distalen Ende des Radius, die als Lager- bzw. Trägerschale ausgebildet ist, deren proximale Seite Verankerungselemente umfasst, und deren distale Seite wenigstens eine konkave Lagerfläche definiert, sowie mit einer karpalen Komponente gelöst, die bezüglich der Radialkomponente einerseits und bezüglich zu, insbesondere allen, der karpalen Komponenten benachbarten, Handwurzelknochen andererseits beweglich gelagert ist.

Ein wesentlicher Punkt der Erfindung liegt darin, dass erfindungsgemäß nur die Radialkomponente am Radius befestigt werden muss, während die karpale Komponente bezüglich der Radialkomponente einerseits sowie bezüglich der Handwurzelknochen, weiche der karpale Komponente benachbart sind, andererseits, beweglich gelagert ist. Somit ist eine Fixierung der karpalen Komponente an Handwurzelknochen nicht notwendig und insbesondere nicht vorgesehen, so dass eine aufwendige Fixierung einer distalen Handgelenkprothesenkomponente, wie beim Stand der Technik, an den Handwurzel- und/oder Mittelhandknochen vermieden werden kann, so dass erfindungsgemäß eine natürliche bindegewebige Stabilisierung der Handwurzelknochen untereinander verbleibt, die Prothese durch den Band- und Bindegewebeapparat der Hand flexibel fixiert und kein Handwurzelknochen durch das Einbringen einer Schraube oder eines Nagels, wie dies beim Stand der Technik notwendig war, geschwächt wird.

Vielmehr ist erfindungsgemäß vorgesehen, dass die karpale Komponente zwischen die Lagerfläche der Radialkomponente und die bestehenden Handwurzelknochen eingebracht wird, oder die dem Radius nächstliegenden Handwurzelknochen, insbesondere das Kahnbein und das Mondbein, ersetzt. Das distale Gelenk zwischen Radius und Ulna (Elle) wird geschont und intakt belassen.

Gemäß einer bevorzugten Ausführungsform der Erfindung erstrecken sich die Verankerungselemente an der proximalen Seite der Radialkomponente in dorsal-palmarer Richtung, d.h. in einer Richtung, die sich vom Handrücken zur Handinnenfläche erstreckt. Entsprechend dieser Richtung der Verankerungselemente ist das distale Ende des Radius für einen Einsatz der Verankerungselemente in dorsal-palmarer Richtung präpariert, so dass eine, insbesondere zementfreie, Implantation der Prothese in dieser dorsal-palmaren Richtung möglich ist.

Zu diesem Zweck umfassen die Verankerungselemente erfindungsgemäß einen, vorzugsweise zwei voneinander beabstandete, sich jeweils dorsal-palmarer Richtung erstreckende Stege, deren freie Enden vorzugsweise verbreitert, insbesondere wulstartig verbreitert sind, wobei die Verbreiterung der Stege im Querschnitt rechteckförmig, trapezförmig, oval oder zylinderförmig ausgebildet ist.

Auf diese Weise ist eine erfindungsgemäße Handgelenkprothese äußerst einfach implantierbar, nämlich über einen dorsalen Zugang im Bereich der dritten Strecksehne, durch welchen die karpale Komponente, nach einem zumeist notwendigen vorherigen Entfernen der körpernahen Handwurzelreihe (proximal row) und Teilen der distalen Handwurzelreihe (distal row), wobei die Resektionsränder entsprechend der Form der karpalen Komponente angepasst werden und ein distales konkaves Lager in den Handwurzel- und/oder Mittelhandknochen vorbereitet wird., sowie die Radialkomponente der erfindungsgemäßen Handgelenkprothese einfach von oben in das Handgelenk eingeschoben, respektive dieses ersetzen kann, wobei die Verankerungselemente in entsprechende Ausnehmungen, die im distalen Ende des Radius präpariert sind, von dorsal nach palmar eingeschoben und darin verankert werden.

Eine gesonderte Fixierung der karpalen Komponente nach einer Implantation ist erfindungsgemäß nicht notwendig, da die implantierte karpale Komponente, ähnlich wie die übrigen Handwurzelknochen sowie dieursprünglichen, entfernten Mittelhand- und Handwurzelknochen, durch die Bänder- und Bindegewebsstruktur der Hand gestützt und korrekt an dem vorgesehenen Ort gehalten wird. Erfindungsgemäß ist die karpale Komponente somit zwischen der konkaven Lagerfläche der Radialkomponente und den verbliebenen Handwurzelknochen in beweglicher Weise fixiert, so dass die Beweglichkeit des Handgelenks weitestgehend der natürlichen Beweglichkeit entspricht und auch nach einer Implantation der erfindungsgemäßen Handgelenkprothese erhalten bleibt.

Erfindungsgemäß ist die karpale Komponente als länglich ovaler, im Wesentlichen ei- oder rugbyballförmiger Körper ausgebildet, der an der konkaven Lagerfläche der Radialkomponente einerseits sowie an den verbliebenen Handwurzelknochen andererseits anliegt und gegenüber den jeweiligen Anlagepunkten beweglich ist, so dass insbesondere eine Palmarflexion sowie eine Dorsalextension, jedoch auch eine Radialabduktion und eine Ulnarabduktion ohne Beeinflussung der Beweglichkeit im distalen Radioulnargelenk möglich sind. Dies wird erfindungsgemäß durch die ellipsoid-, eioder rugbyballförmige Gestalt der karpalen Komponente ermöglicht.

Alternativ kann die karpale Komponente auch als länglich ovaler, im Wesentlichen eioder rugbyballförmiger Körper ausgebildet sein, der wenigstens eine Einschnürung aufweist, die vorzugsweise im Wesentlichen orthogonal zu seiner Längsachse verläuft, so dass die karpale Komponente alternativ einen im Wesentlichen hantel- oder erdnusshülsenförmigen Körper definiert.

Diese hantel- oder erdnusshülsenförmige Gestaltung der karpale Komponente weist diverse Vorteile auf, die zum einen darin bestehen, dass die jeweiligen, der Einschnürung jeweils benachbarten Abschnitte der karpale Komponente unterschiedlich groß, d.h. mit unterschiedlichen Ellipsenradien ausgebildet sein können, so dass im Falle des Ersatzes von Handwurzel- und/oder Mittelhandknochen durch die karpale Komponente einer individuellen Größe der jeweiligen Handwurzelknochen und der karpalen Höhe Rechnung getragen werden kann, so dass die erfindungsgemäße Handgelenkprothese sehr paßgenau an den jeweiligen Träger anpassbar ist. Darüber hinaus ermöglicht die hantel- oder erdnusshülsenförmige Ausgestaltung der karpalen Komponente einen optimierten Halt in der wenigstens einen konkaven Lagerfläche der Radialkomponente, so dass nach einer Implantation der Handgelenkprothese eine optimierte Bewegungsführung des Prothesengelenks möglich ist, die der natürlichen Streck- und Beugebeweglichkeit eines Handgelenks sehr nahe kommt und nahezu vollständig entspricht.

Gemäß einer weiteren Ausführungsform der Erfindung ist zwischen der Radialkomponente und der karpalen Komponente ein, beispielsweise im Wesentlichen flächiger, gegebenenfalls mit wenigstens einer löffelartigen Fläche versehener, Zwischenelementkörper vorgesehen, der mit seiner proximalen Seite mit der distalen Seite der Radialkomponente und mit seiner distalen Seite mit der proximalen Seite der karpalen Komponente zusammenwirkt. Die Vorsehung eines solchen Zwischenelementkörpers bringt erfindungsgemäß zahlreiche Vorteile mit sich, wobei an erster Stelle erwähnt sei, dass ein solcher Zwischenelementkörper erstens zur Vergrößerung der Liegefläche der karpalen Komponente an der Radialkomponente dient und zweitens das Ausmaß an Beweglichkeit in Extension, Flexion, vor allem aber auch in radioulnarer Richtung erhöht, ferner als Ausgleichselement zwischen Radialkomponente und karpaler Komponente der erfindungsgemäßen Handgelenkprothese dienen kannund der Zwischenelementkörper im Übrigen hinsichtlich seiner proximal-distalen Erstreckung optimal an die zu implantierende Handgelenkprothese angepaßt werden kann, so dass ein optimierter Sitz der implantierten Handgelenkprothese sowie eine optimierte Halterung der karpalen Komponente möglich ist.

Es sei an dieser Stelle betont, dass auch der Zwischenelementkörper ohne zusätzliche Befestigung, insbesondere ohne zusätzliche Befestigung durch Schrauben, Nägel oder Klammern implantiert wird und, entsprechend der erfindungsgemäßen karpalen Komponente rein durch die Gelenkspannung sowie durch den Bänder-Bindegewebs- und Muskelapparat sowie gegebenenfalls eine stabilisierende Vernarbung der Hand gehalten wird.

Ferner ist erfindungsgemäß distal der karpalen Komponente eine, gegebenenfalls mit wenigstens einer proximal angeordneten löffelartigen Fläche versehene, distal-karpale Komponente vorgesehen, die mit ihrer proximalen Seite mit der distalen Seite der karpalen Komponente und mit ihrer distalen Seite mit Handwurzel- und/oder Mittelhandknochen zusammenwirkt und optional mit Handwurzel- und/oder Mittelhandknochen, insbesondere fest, verbunden ist.

Diese distal-karpale Komponente kann, beispielsweise bei einer labilen Bindegewebs-und Bänderstruktur der Hand zusätzliche Stabilität verleihen, indem die distal-karpale Komponente mit ihrer distalen Seite an Handwurzel- und/oder Mittelhandknochen anliegt und mit diesen in Wirkeingriff steht und/oder mit diesen Handwurzel- und/oder Mittelhandknochen, insbesondere fest, verbunden ist. An der proximalen Seite dieser distal-karpalen Komponente ist diese gegenüber der distalen Seite der karpalen Komponente beweglich gelagert, so dass eine gegenüber dem bisherigen Stand der Technik optimierte Beweglichkeit auch bei Vorsehung einer solchen distal-karpalen Komponente optimal gegeben ist. Die proximale Seite der distal-karpalen Komponente ist hierbei so ausgeformt, dass sie wenigstens eine konkave Lagerfläche definiert, die mit dem länglich ovalen, im wesentlichen ei- oder rugbyballförmigen Körper der karpalen Komponente führend zusammenwirkt.

Im Übrigen ist/sind wenigstens eine Kombination von zwei folgender Elemente, nämlich Radialkomponente, Zwischenelementkörper, karpale Komponente und distal-karpale Komponente, gegebenenfalls unter Einschluss wenigstens eines weiteren der Elemente miteinander verbunden und/oder geführt, wobei die Verbindung und/oder Führung, mittels einem oder mehreren der folgenden Einrichtungen, die ausgewählt sind aus Fäden, Bändern, Drähten, Scharnieren, insbesondere Filmscharnieren oder flexiblen Stiftscharnieren, magnetischer Wechselwirkung, Auskragungs-Ausnehmungs-Kombination, beispielsweise in Form von ineinandergreifenden Strukturen, realisiert ist/sind.

Durch eine Verbindung der vorgenannten Komponenten, nämlich der Radialkomponente, des Zwischenelementkörpers, der karpalen Komponente sowie der distal-karpalen Komponente untereinander kann der erfindungsgemäßen Handgelenksprothese nach Wunsch eine zusätzliche Stabilität verliehen werden, ohne jedoch die Beweglich der erfindungsgemäßen Handgelenkprothese, insbesondere der implantierten Handgelenkprothese einzuschränken. Diese Verbindungs- und/oder Führungseinrichtungen sind für die erfindungsgemäße Handgelenkprothese optional, d.h. nicht zwingend notwendig, und dienen im Wesentlichen einer ergänzenden Stabilisierung sowie, nach Bedarf, auch Führung des Handgelenks, sofern der köpereigene Bänder-Bindegewebs- und Muskelapparat nicht ausreichend in der Lage ist, das Handgelenk zu stabilisieren.

Des Weiteren sei darauf hingewiesen, dass zumindest die Lagerfläche der Radialkomponente sowie gegebenenfalls die Fläche des Zwischenelementkörpers und/oder der distal-karpalen Komponente in Draufsicht, d.h. in einer Ansicht von distal nach proximal, oval oder ellipsoid ausgebildet sind und/oder die Lagerfläche oder Fläche Teil einer Ellipsoid-, Hyperboloid- oder Paraboloidfläche sind. Auf diese Weise kann die erfindungsgemäße Handgelenkprothese in optimierter funktioneller Weise an ein natürliches Handgelenk angepaßt sein und in optimierter Weise an das distale Ende des Radius angesetzt werden, wobei die konkave Lagerfläche der Radialkomponente oder eine für die karpale Komponente entsprechend geformte Anlagefläche des Zwischenelementkörpers einen paßgenauen Sitz der karpalen Komponente an der jeweiligen Lagerfläche der Radialkomponente oder Anlagefläche des Zwischenelementkörpers oder Anlagefläche der distal-karpalen Komponente gewährleistet.

Je nach gewünschter Form der karpalen Komponente weist die konkave Lagerfläche und/oder eine der karpalen Komponente zugewandte Anlageseite des Zwischenelementkörpers oder eine Anlagefläche der distal-karpalen Komponente wenigstens eine konkave Fläche, nämlich im Falle eines ei- oder rugbyballförmigen karpalen Komponentenkörpers, oder aber eine doppelkonkave Lagerfläche oder Anlagefläche, nämlich für den Fall eines hantel- oder erdnusshülsenförmigen karpalen Komponentenkörpers, auf, die sich in Form von zwei inneren Kugelflächen mit unterschiedlichem Radius darstellen.

Gemäß einer weiteren Ausführungsform ist die proximale Seite des Zwischenelementkörpers im Wesentlichen komplementär zur distalen Seite der Radialkomponente, insbesondere formschlüssig mit dieser und/oder die proximale Seite der distal-karpalen Komponente (70) im Wesentlichen komplementär zur distalen Seite der karpalen Komponente (30), insbesondere formschlüssig mit dieser, ausgebildet. Auf diese Weise wird gewährleistet, dass der Zwischenelementkörper einen sicheren, verrutschfreien Sitz an der distalen Seite der Radialkomponente hat, wobei die jeweilige Lagerfläche der Radialkomponente sowie die proximale Seite des Zwischenelementkörpers jeweils komplementär profiliert sein können, wobei die jeweiligen Profilierungen ineinander greifen und eine Lagedefinition zwischen proximaler Seite des Zwischenelementkörpers und der Lagerfläche der Radialkomponente bewirken, wobei betont sei, dass in einem solchen Fall eine konkave Ausgestaltung der Lagerfläche der Radialkomponente nicht zwingend notwendig ist, nämlich dann, wenn zumindest die der karpalen Komponente zugewandte distale Seite des Zwischenelementkörpers eine solche konkave Fläche aufweist, die einerseits eine sichere Fixierung der karpalen Komponente, jedoch auch eine gute Bewegbarkeit der karpalen Komponente an der konkaven Fläche, nämlich im Sinne einer beidseitigen Beugbarkeit als auch Abspreizbarkeit des Handgelenks nach erfolgter Implantation gewährleistet. Dieselbe Maßnahmen sind analog auch zwischen der distal-karpalen Komponente und der karpalen Komponente möglich, um eine optimierte Führung zwischen diesen beiden Elementen bei gleichzeitiger optimierter Beweglichkeit des implantierten erfindungsgemäßen Handgelenks zu gewährleisten.

Erfindungsgemäß ist die Radialkomponente und/oder die karpale Komponente und/oder der Zwischenelementkörper und/oder die distal-karpale Komponente sowie Wirkoberflächen zwischen der Radialkomponente, der karpalen Komponente, der distal-karpalen Komponente und dem Zwischenelementkörper aus einem oder mehreren unter physiologischen Bedingungen inerten Materialien, insbesondere ausgewählt aus nachfolgender Auswahl, hergestellt: Kunststoff, bevorzugt Polyethylen (PE), besonders bevorzugt insbesondere vernetztes ultrahochmolekulares Polyethylen (UHMWPE) mit einem Molekulargewicht von größer 1000000, Kunststoff-Faserverbund, Polyetheretherketon (PEEK), Keramik, Pyrocarbon, Metall, bevorzugt Titan oder eine Titan-und/oder Kobaltchromlegierung, Edelmetall und/oder eine Edelmetaillegierung, sowie Kombinationen der vorgenannten Materialien.

Die Verwendung von Keramik oder Metalllegierungen bietet den Vorteil eines extrem geringen, bzw. nicht vorhandenen Abriebs im Gebrauch der Handgelenkprothese, während die Verwendung von Kunststoff, insbesondere hochvernetztem ultrahochmolekularem Polyethylen (UHMWPE) Dämpfungseigenschaften mit sich bringt und ein solcher Kunststoff besonders leicht zu bearbeiten ist. Somit kann sowohl die gesamte Prothese als auch einzelne Prothesenteile aus Kunststoff gefertigt sein, wobei, je nach Anwendungsfall, auch Keramik oder Metall, insbesondere Titan, ein bevorzugtes Material zur Herstellung vorgenannter Komponenten darstellt.

Des weiteren sei darauf hingewiesen, dass die proximale Seite der Radialkomponente, sowie insbesondere die Verankerungselemente aufgeraut und/oder und/oder porös und/oder mit einem Porennetzwerk ausgebildet sowie ferner insbesondere mit Hydroxylapatit beschichtet sind, um ein Einwachsen von Osteoplasten ausgehend vom distalen Ende des Radius an bzw, in die proximale Seite der Radialkomponente zu ermöglichen, um auf diese Weise neben den Verankerungselementen zu einer optimierten Fixierung und Befestigung der Radialkomponente am distalen Ende des Radius beizutragen.

Weiter Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen:
- Fig. 1: eine schematische Darstellung einer ersten Ausführungsform einer erfindungsgemäßen Handgelenkprothese;
- Fig. 2 bis 5: schematische Darstellungen einer erfindungsgemäßen Handgelenkprothese gemäß Fig. 1 in am Radius fixiertem Zustand in unterschiedlichen Ansichten;
- Fig. 6 und 7: eine schematische Darstellung einer zweiten Ausführungsform einer erfindungsgemäßen Handgelenkprothese in Dorsal- bzw. Palmaransicht;
- Fig. 8 bis 12: schematische Darstellungen einer weiteren Ausführungsform einer erfindungsgemäßen Handgelenkprothese in an das distale Ende eines Radius angesetztem Zustand in unterschiedlichen Ansichten;
- Fig. 13: eine schematische Darstellung einer erfindungsgemäßen Handgelenkprothese gemäß den Figuren 8 bis 12 ohne karpale Komponente; und
- Fig. 14: eine schematische Ansicht eines zur Implantation präparierten distalen Endes eines Radius.

In der nachfolgenden Beschreibung werden für gleiche und gleich wirkende Teile dieselben Bezugsziffern verwendet.

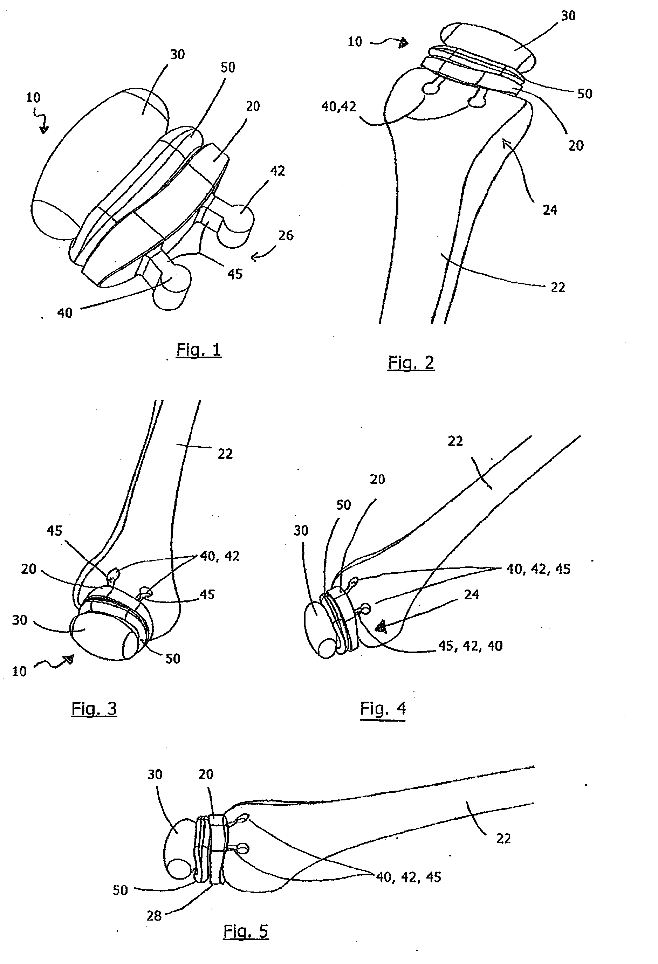

Fig. 1 zeigt eine schematische Darstellung einer ersten Ausführungsform einer erfindungsgemäßen Handgelenkprothese 10. Die Handgelenkprothese 10 weist eine Radialkomponente 20 mit Verankerungselementen 40, 42 zur Befestigung am distalen Ende eines Radius auf. Die Verankerungselemente 40, 42 sind als Stege 45 mit einer im Querschnitt runden Verbreiterung am freien Ende der Stege 45 ausgebildet. Die Verankerungselemente 40, 42 sind an der proximalen Seite 26 der Radialkomponente 20 angeordnet. Auf der distalen Seite der Radialkomponente 20 befindet sich ein flächiger, kissenartig ausgebildeter Zwischenelementkörper, der mit seiner proximalen Seite an der konkaven Lagerfläche der Radialkomponente 20 anliegt. Auf der distalen, ebenfalls leicht konkav ausgebildeten Seite des Zwischenelementkörpers 50 befindet sich ein länglich ovaler, im Wesentlichen ei- bzw. rugbyballförmiger karpaier Komponentenkörper.

Die Figuren 2 bis 5 zeigen jeweils unterschiedliche Ansichten der erfindungsgemäßen Handgelenkprothese gemäß Fig. 1 in an das distale Ende des Radius an- bzw. eingesetztem Zustand, wobei die Verankerungselemente 40, 42 der Handgelenkprothese 10 in Richtung von dorsal nach palmar in entsprechende präparierte Ausnehmungen am distalen Ende 24 des Radius 22 eingesetzt und dort fixiert sind und ulnar das distale Radioulnargelenk nicht tangieren.

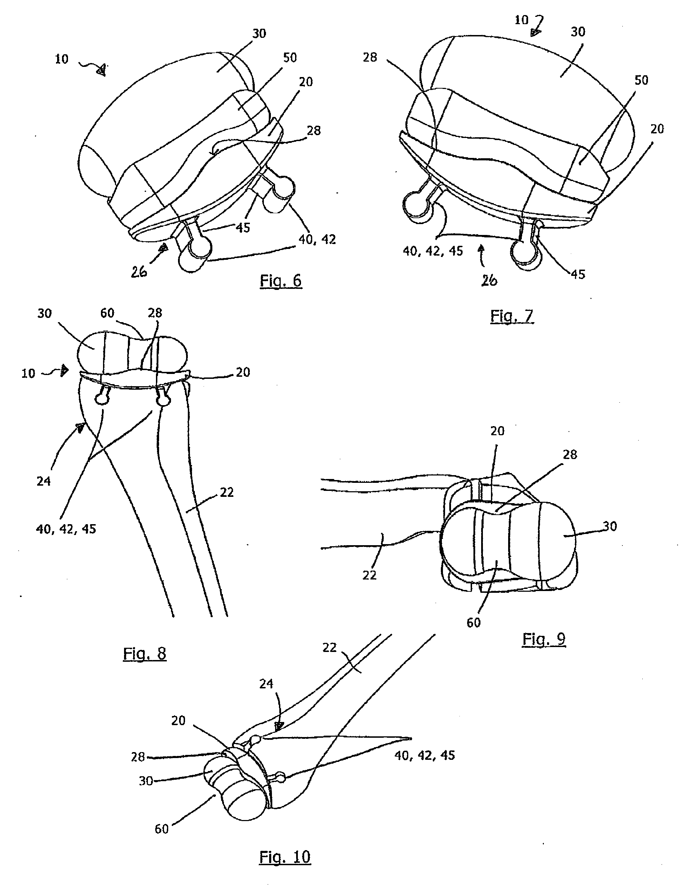

Die Figuren 6 und 7 zeigen jeweils schematische Ansichten einer zweiten Ausführungsform einer erfindungsgemäßen Handgelenkprothese, jeweils in dorsaler bzw. palmarer Ansicht. Gemäß dieser Ausführungsform ist die Lagerfläche 28 der Radialkomponente mit zwei konkaven Lagerflächen versehen, die durch eine rippenartige Erhöhung voneinander getrennt sind. Auf dieser Lagerfläche 28 befindet sich ein auf seiner proximalen Seite komplementär zur Lagerfläche 28 ausgebildeter Zwischenelementkörper 50, der paßgenau an der Lagerfläche 28 anliegt. Auf der distalen Seite des Zwischenelementkörpers befindet sich eine karpale Komponente, die als länglich ovaler, im wesentlichen ei- oder rugbyballförmiger Körper ausgebildet ist.

Die weiteren Figuren 8 bis 12 zeigen jeweils schematische Ansichten einer erfindungsgemäßen Handgelenkprothese, wobei die Radialkomponente 20 derjenigen aus den Figuren 6 und 7 entspricht. Auf der Lagerfläche 28 der Radialkomponente 20 ist unmittelbar eine im wesentlichen hantel- bzw. erdnusshülsenförmige karpale Komponente angeordnet, wobei die karpale Komponente eine Einschnürung 60 aufweist, die sich im Wesentlichen rund um die karpale Komponente, und zwar im wesentlichen orthogonal zur Längsachse der karpalen Komponente 30 erstreckt, Die beiden Teilabschnitte der karpalen Komponente 30, die sich auf den jeweiligen Seiten der Einschnürung 60 befinden, weisen unterschiedliche Ellipsenradien auf, so dass sie optimal an die unterschiedlich konkaven Lagerflächen 28 der Radialkomponente 20 angepaßt sind.

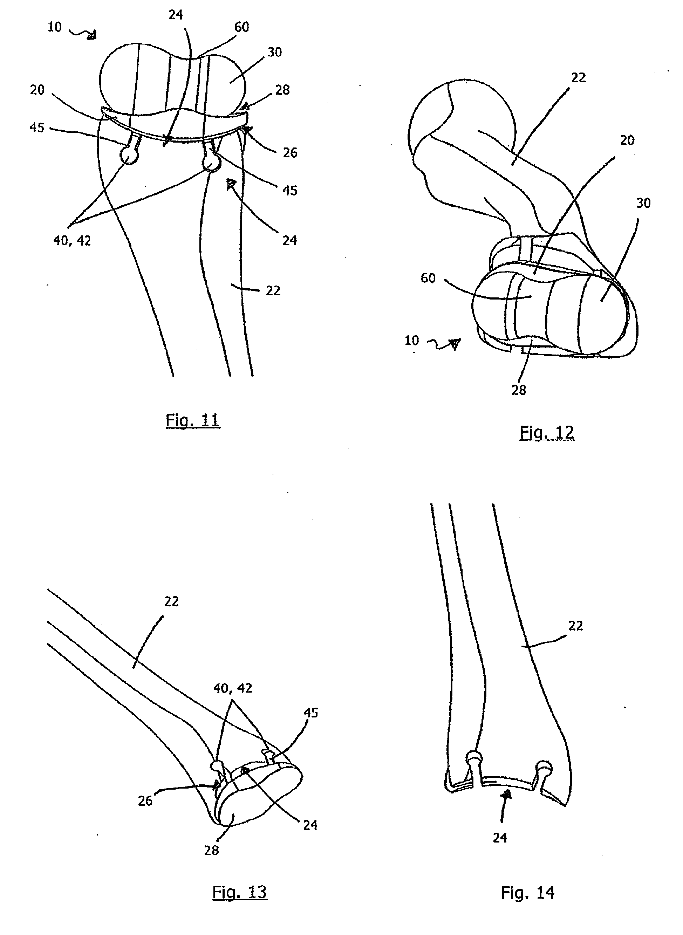

Fig. 13 zeigt eine weitere schematische Darstellung einer erfindungsgemäßen Radialkomponente 20 in an das distale Ende 24 des Radius 22 angesetztem Zustand, jedoch ohne karpale Komponente und ohne Zwischenelementkörper, so dass die doppelkonkave Lagerfläche 28 gut erkennbar ist, wobei die jeweiligen Konkavflächen durch eine wellenartige Rippe voneinander getrennt sind.

Fig. 14 zeigt das distale Ende 24 eines zur Implantation vorbereiteten Radius 22 mit für die Verankerungselemente vorgesehenen Ausnehmungen.

### Bezugszeichenliste

- 10: Handgelenkprothese
- 20: Radialkomponente
- 22: Radius
- 24: distales Ende des Radius
- 26: proximale Seite der Radialkomponente
- 28: Lagerfläche
- 30: karpale Komponente
- 40: Verankerungselement
- 42: Verankerungselement
- 45: Steg
- 50: Zwischenelementkörper
- 60: Einschnürung
- 70: distal-karpale Komponente

## Patentansprüche

1. Handgelenkprothese (10) mit einer Radialkomponente (20) zur Befestigung am distalen Ende (24) des Radius (22), die als Lager- bzw. Trägerschale ausgebildet ist, deren proximale Seite (26) Verankerungselemente (40, 42) umfasst, und deren distale Seite wenigstens eine konkave Lagerfläche (28) definiert, sowie mit einer karpalen Komponente (30), die bezüglich der Radiaikomponente (20) einerseits beweglich gelagert ist; **dadurch gekennzeichnet, dass** die Karpale Komponente (30) bezüglich zu, insbesondere allen, der karpalen Komponente (30) benachbarten Handwurzelknochen andererseits bewegüch gelagert ist.

2. Handgelenkprothese nach Anspruch 1,
**dadurch gekennzeichnet, dass**
die karpale Komponente (30) als länglich ovaler, im Wesentlichen ei- oder rugbyballförmiger Körper oder alternativ als solcher Körper mit wenigstens einer Einschnürung (60), die vorzugsweise im Wesentlichen orthogonal zu seiner Längsachse verläuft, ausgebildet ist, so dass die karpale Komponente (30) alternativ einen im Wesentlichen hantel- oder erdnusshülsenförmigen Körper definiert.

3. Handgelenkprothese nach einem der vorhergehenden Ansprüche,
**dadurch gekennzeichnet, dass**
zwischen der Radialkomponente (20) und der karpalen Komponente (30) ein, beispielsweise im Wesentlichen flächiger, gegebenenfalls mit wenigstens einer löffelartigen Fläche versehener, Zwischenelementkörper (50) vorgesehen ist, der mit seiner proximalen Seite mit der distalen Seite der Radialkomponente (20) und mit seiner distalen Seite mit der proximalen Seite der karpalen Komponente (30) zusammenwirkt.

4. Handgelenkprothese nach einem der vorhergehenden Ansprüche,
**dadurch gekennzeichnet, dass**
distal der karpalen Komponente (30) eine, gegebenenfalls mit wenigstens einer proximal angeordneten löffelartigen Fläche versehene, distal-karpale Komponente (70) vorgesehen ist, die mit ihrer proximalen Seite mit der distalen Seite der karpalen Komponente (30) und mit ihrer distalen Seite mit Handwurzel- und/oder Mittelhandknochen zusammenwirkt und optional mit Handwurzelund/oder Mittelhandknochen, insbesondere fest, verbunden ist.

5. Handgelenkprothese nach einem der vorhergehenden Ansprüche,
**dadurch gekennzeichnet, dass**
wenigstens eine Kombination von zwei folgender Elemente, nämlich Radialkomponente (20), Zwischenelementkörper (50), karpale Komponente (30) und distal-karpale Komponente (70), gegebenenfalls unter Einschluss wenigstens eines weiteren der Elemente miteinander verbunden und/oder geführt, ist/sind, wobei die Verbindung und/oder Führung, mittels einem oder mehreren der folgenden Einrichtungen, die ausgewählt sind aus Fäden, Bändern, Drähten, Scharnieren, insbesondere Filmscharnieren, magnetischer Wechselwirkung, Auskragungs-Ausnehmungs-Kombination, realisiert ist/sind.

6. Handgelenkprothese nach einem der vorhergehenden Ansprüche,
**dadurch gekennzeichnet, dass**
die Verankerungselemente (40, 42) sich in Richtung dorsal/palmar (D-P) erstrecken, so dass insbesondere bei zementfreier Implantation der Prothese (10) diese in dorsal/palmarer (D-P) Richtung einbringbar ist.

7. Handgelenkprothese nach einem der vorhergehenden Ansprüche,
**dadurch gekennzeichnet, dass**
die Verankerungselemente (40, 42) einen, vorzugsweise zwei voneinander beabstandete, sich jeweils dorsal/palmar erstreckende Stege (45) umfassen, deren freie Enden vorzugsweise verbreitert, insbesondere wulstartig verbreitert, sind.

8. Handgelenkprothese nach Anspruch 7,
**dadurch gekennzeichnet, dass**
die Verbreiterung der Stege (45) im Querschnitt rechteckförmig, trapezförmig, oval oder zylinderförmig ausgebildet ist.

9. Handgelenkprothese nach einem der vorhergehenden Ansprüche 3 bis 8,
**dadurch gekennzeichnet, dass**
zumindest die Lagerfläche (28) der Radialkomponente (20), sowie die Fläche des Zwischenelementkörpers (50) und/oder der distal-karpalen Komponente (70) in Draufsicht oval oder ellipsoid ausgebildet und/oder die Lagerfläche (28) oder Fläche Teil einer Ellipsoid-, Hyperboloid- oder Paraboloidfläche sind.

10. Handgelenkprothese nach einem der vorhergehenden Ansprüche 3 bis 9,
**dadurch gekennzeichnet, dass**
die konkave Lagerfläche (28) und/oder der Zwischenelementkörper (50) und/oder die distal-karpale Komponente (70) wenigstens eine Kugelfläche, insbesondere wenigstens zwei Kugelflächen mit unterschiedlichem Radius umfasst/umfassen.

11. Handgelenkprothese nach einem der vorhergehenden Ansprüche 3 bis 10,
**dadurch gekennzeichnet, dass**
die proximale Seite des Zwischenelementkörpers (50) im Wesentlichen komplementär zur distalen Seite der Radialkomponente (20), insbesondere formschlüssig mit dieser und/oder die proximale Seite der distal-karpalen Komponente (70) im Wesentlichen komplementär zur distalen Seite der karpalen Komponente (30), insbesondere formschlüssig mit dieser, ausgebildet ist.

12. Handgelenkprothese nach einem der vorhergehenden Ansprüche 3 bis 11,
**dadurch gekennzeichnet, dass**
die Radialkomponente (20) und/oder die karpale Komponente (30) und/oder der Zwischenelementkörper (50) und/oder die distal-karpale Komponente (70) sowie Wirkoberflächen zwischen der Radialkomponente (20), der karpalen Komponente (30), der distal-karpale Komponente (70) und dem Zwischenelementkörper (50) aus einem oder mehreren unter physiologischen Bedingungen inerten Materialien, insbesondere ausgewählt aus nachfolgender Auswahl hergestellt sind: Kunststoff, bevorzugt Polyethylen (PE), besonders bevorzugt insbesondere vernetztes ultrahochmolekulares Polyethylen (UHMWPE) mit einem Molekulargewicht von größer 1000000, Kunststoff-Faserverbund, Polyetheretherketon (PEEK), Keramik, Pyrocarbon, Metall, bevorzugt Titan oder eine Titan- und/oder Kobaltchromlegierung, Edelmetall und/oder eine Edelmetalllegierung, sowie Kombinationen der vorgenannten Materialien.

13. Handgelenkprothese nach einem der vorhergehenden Ansprüche,
**dadurch gekennzeichnet, dass**
die proximale Seite (26) der Radialkomponente (20) sowie insbesondere die Verankerungselemente (40, 42) und/oder die distale Seite der distal-karpalen Komponente (70) aufgeraut und/oder porös und/oder mit einem Porennetzwerk ausgebildet sowie insbesondere mit Hydroxylapatit beschichtet sind.

## Claims

1. Wrist prosthesis (10) having a radial component (20) for attachment to the distal end (24) of the radius (22), the radial component (20) being in the form of a bearing shell or carrier shell the proximal side (26) of which comprises anchoring elements (40, 42) and the distal side of which defines at least one concave bearing surface (28), and having a carpal component (30) which is, for the first part, mounted so as to be movable relative to the radial component (20);
**characterised in that**
the carpal component (30) is, for the other part, mounted so as to be movable relative to bones of the carpus that are adjacent to the carpal component (30), especially all such bones.

2. Wrist prosthesis according to claim 1,
**characterised in that**
the carpal component (30) is in the form of an elongately oval, substantially egg-shaped or rugby-ball-shaped body or alternatively in the form of such a body having at least one constriction (60) which preferably extends substantially orthogonally with respect to the longitudinal axis of the body, so that the carpal component (30) alternatively defines a substantially dumbbell-shaped or peanut-shaped body.

3. Wrist prosthesis according to either one of the preceding claims,
**characterised in that**
between the radial component (20) and the carpal component (30) there is provided an intermediate element body (50), which is, for example, substantially planar and is optionally provided with at least one spoon-like surface, which intermediate element body (50) co-operates, by means of its proximal side, with the distal side of the radial component (20) and, by means of its distal side, with the proximal side of the carpal component (30).

4. Wrist prosthesis according to any one of the preceding claims,
**characterised in that**
distally of the carpal component (30) there is provided a distal-carpal component (70) which is optionally provided with at least one proximally arranged spoon-like surface, which distal-carpal component (70) co-operates, by means of its proximal side, with the distal side of the carpal component (30) and, by means of its distal side, with bones of the carpus and/or metacarpus and is optionally connected, especially fixedly connected, to bones of the carpus and/or metacarpus.

5. Wrist prosthesis according to any one of the preceding claims,
**characterised in that**
at least one combination of two of the following elements, namely radial component (20), intermediate element body (50), carpal component (30) and distal-carpal component (70), optionally including at least one further one of the elements, are mutually connected and/or guided, the connection and/or guidance being realised by means of one or more of the following devices selected from: threads, bands, wires, hinges, especially film hinges, magnetic interaction, projection/recess combination.

6. Wrist prosthesis according to any one of the preceding claims,
**characterised in that**
the anchoring elements (40, 42) extend in the dorsal/palmar (D-P) direction, so that, especially in the case of cementless implantation of the prosthesis (10), the latter can be introduced in the dorsal/palmar direction (D-P).

7. Wrist prosthesis according to any one of the preceding claims,
**characterised in that**
the anchoring elements (40, 42) comprise a linker (45), preferably two linkers (45) spaced apart from one another, each extending in the dorsal/palmar direction, the free ends of which preferably have a broadened portion, especially a bulge-like broadened portion.

8. Wrist prosthesis according to claim 7,
**characterised in that**
the broadened portion of the linkers (45) is rectangular, trapezoidal, oval or cylindrical in cross-section.

9. Wrist prosthesis according to any one of preceding claims 3 to 8,
**characterised in that**
at least the bearing surface (28) of the radial component (20), and the surface of the intermediate element body (50) and/or of the distal-carpal component (70) is oval or ellipsoidal in plan view and/or the bearing surface (28) or surface is part of an ellipsoidal, hyperboloidal or paraboloidal surface.

10. Wrist prosthesis according to any one of preceding claims 3 to 9,
**characterised in that**
the concave bearing surface (28) and/or the intermediate element body (50) and/or the distal-carpal component (70) comprise(s) at least one spherical surface, especially at least two spherical surfaces of different radius.

11. Wrist prosthesis according to any one of preceding claims 3 to 10,
**characterised in that**
the proximal side of the intermediate element body (50) is substantially complementary to the distal side of the radial component (20), especially forming a shape-based connection therewith, and/or the proximal side of the distal-carpal component (70) is substantially complementary to the distal side of the carpal component (30), especially forming a shape-based connection therewith.

12. Wrist prosthesis according to any one of preceding claims 3 to 11,
**characterised in that**
the radial component (20) and/or the carpal component (30) and/or the intermediate element body (50) and/or the distal-carpal component (70), as well as effective surfaces between the radial component (20), the carpal component (30), the distal-carpal component (70) and the intermediate element body (50), are made from one or more materials that are inert under physiological conditions, especially selected from the following: plastics, preferably polyethylene (PE), especially preferably crosslinked ultra-high molecular weight polyethylene (UHMWPE) having a molecular weight greater than 1,000,000, plastics/fibre composite, polyether ether ketone (PEEK), ceramics, pyrocarbon, metal, preferably titanium or a titanium and/or cobalt chromium alloy, noble metal and/or a noble metal alloy, and also combinations of the afore-mentioned materials.

13. Wrist prosthesis according to any one of the preceding claims,
**characterised in that**
the proximal side (26) of the radial component (20) and especially the anchoring elements (40, 42) and/or the distal side of the distal-carpal component (70) are roughened and/or porous and/or have a network of pores and are especially coated with hydroxylapatite.

## Revendications

1. Prothèse de poignet (10), comprenant un composant radial (20), destiné à être fixé à l'extrémité distale (24) du radius (22), qui est façonné sous la forme d'un plateau d'appui ou de support dont le côté proximal (26) comporte des éléments d'ancrage (40, 42) et dont le côté distal définit au moins une surface d'appui concave (28), ainsi qu'un composant carpien (30) qui, d'une part, est monté de manière mobile par rapport au composant radial (20), **caractérisée en ce que**, d'autre part, le composant carpien (30) est monté de manière mobile par rapport aux os du carpe, en particulier par rapport à tous les os du carpe adjacents au composant carpien (30).

2. Prothèse de poignet selon la revendication 1, **caractérisée en ce que** le composant carpien (30) est façonné sous la forme d'un corps ovale oblong, sensiblement en forme d'oeuf ou de ballon de rugby ou, en variante, sous la forme d'un corps qui présente au moins un rétrécissement (60), qui de préférence s'étend perpendiculairement à son axe longitudinal, de sorte que le composant carpien (30) définit en variante un corps sensiblement en forme d'haltère ou de coque de cacahuète.

3. Prothèse de poignet selon l'une quelconque des revendications précédentes, **caractérisée en ce qu'**un corps d'élément intermédiaire (50), par exemple sensiblement plan, doté le cas échéant d'au moins une surface en forme de cuillère, qui coopère par son côté proximal avec le côté distal du composant radial (20) et par son côté distal avec le côté proximal du composant carpien (30) est prévu entre le composant radial (20) et le composant carpien (30).

4. Prothèse de poignet selon l'une quelconque des revendications précédentes, **caractérisée en ce qu'**un composant carpien distal (70), doté le cas échéant d'au moins une surface en forme de cuillère disposée du côté proximal, qui coopère par son côté proximal avec le côté distal du composant carpien (30) et par son côté distal avec les os du carpe et/ou du métacarpe et qui est facultativement relié, en particulier fixement, aux os du carpe et/ou du métacarpe est prévu du côté distal par rapport au composant carpien (30).

5. Prothèse de poignet selon l'une quelconque des revendications précédentes, **caractérisée en ce qu'**au moins une combinaison de deux éléments consécutifs, notamment le composant radial (20), le corps d'élément intermédiaire (50), le composant carpien (30) et le composant carpien distal (70), incluant le cas échéant au moins un autre de ces éléments, sont reliés et/ou guidés ensemble, la liaison et/ou le guidage étant réalisé au moyen d'un ou plusieurs dispositifs choisis parmi des fils textiles, des bandes, des fils métalliques, des charnières, en particulier des charnières film, une interaction magnétique et une combinaison creux/saillie.

6. Prothèse de poignet selon l'une quelconque des revendications précédentes, **caractérisée en ce que** les éléments d'ancrage (40, 42) s'étendent dans le sens dorso-palmaire (D-P), de sorte que, lorsqu'elle est implantée sans ciment, la prothèse (10) peut être introduite dans le sens dorso-palmaire (D-P).

7. Prothèse de poignet selon l'une quelconque des revendications précédentes, **caractérisée en ce que** les éléments d'ancrage (40, 42) comprennent une, de préférence, deux ailettes (45), espacées l'une de l'autre, s'étendant chacune dans le sens dorso-palmaire, dont les extrémités libres sont de préférence élargies, en particulier en forme de bourrelet.

8. Prothèse de poignet selon la revendication 7, **caractérisée en ce que** l'élargissement des ailettes (45) est façonné, en coupe transversale, sous forme rectangulaire, trapézoïdale, ovale ou cylindrique.

9. Prothèse de poignet selon l'une quelconque des revendications 3 à 8 précédentes, **caractérisée en ce que**, vues de dessus, au moins la surface d'appui (28) du composant radial (20) ainsi que la surface du corps d'élément intermédiaire (50) et/ou du composant carpien distal (70) sont façonnés sous forme ovale ou ellipsoïdale et/ou **en ce que** la surface d'appui (28) ou la surface forme une partie d'une surface ellipsoïdale, hyperboloïdale ou paraboloïdale.

10. Prothèse de poignet selon l'une quelconque des revendications 3 à 9 précédentes, **caractérisée en ce que** la surface d'appui concave (28) et/ou le corps d'élément intermédiaire (50) et/ou le composant carpien distal (70) comportent au moins une, en particulier au moins deux surfaces sphériques de rayons différents.

11. Prothèse de poignet selon l'une quelconque des revendications 3 à 10 précédentes, **caractérisée en ce que** le côté proximale du corps d'élément intermédiaire (50) est sensiblement complémentaire au côté distal du composant radial (20), en particulier est façonné par complémentarité de forme avec celle-ci, et/ou **en ce que** le côté proximal du composant carpien distal (70) est sensiblement complémentaire au côté distal du composant carpien (30), en particulier par complémentarité de forme avec celle-ci.

12. Prothèse de poignet selon l'une quelconque des revendications 3 à 11 précédentes, **caractérisée en ce que** le composant radial (20) et/ou le composant carpien (30) et/ou le corps d'élément intermédiaire (50) et/ou le composant carpien distal (70), ainsi que les surfaces actives entre le composant radial (20), le composant carpien (30), le composant carpien distal (70) et le corps d'élément intermédiaire (50) sont fabriquées à partir d'un ou plusieurs matériaux inertes dans des conditions physiologiques, en particulier choisis parmi la sélection suivante : matière plastique, de préférence le polyéthylène (PE), de façon particulièrement préférée, en particulier, le polyéthylène de très haut poids moléculaire (UHMWPE) dont le poids moléculaire est supérieur à 1 000 000, composite plastique/fibres, polyétheréthercétone (PEEK), céramique, carbone pyrolytique, métal, de préférence le titane ou un alliage titane-chrome et/ou cobalt-chrome, un métal précieux et/ou un alliage de métaux précieux, ainsi que des combinaisons des matériaux précités.

13. Prothèse de poignet selon l'une quelconque des revendications précédentes, **caractérisée en ce que** le côté proximal (26) du composant radial (20), ainsi qu'en particulier les éléments d'ancrage (40, 42) et/ou le côté distal du composant carpien distal (70) sont rendus rugueux et/ou poreux et/ou façonnés avec un réseau poreux ainsi que, en particulier, revêtus d'hydroxyapatite.
